Europäisches Patentamt

(19) European Patent Office

Office européen des brevets

(11) Numéro de publication : **0 239 485 B1**

(12) # FASCICULE DE BREVET EUROPEEN

(45) Date de publication du fascicule du brevet :
06.11.91 Bulletin 91/45

(51) Int. Cl.$^5$ : **A61F 2/34**

(21) Numéro de dépôt : **87400631.5**

(22) Date de dépôt : **20.03.87**

(54) **Cotyle auto-bloquant pour prothèse cotyloidienne, notamment pour prothèse coxo-fémorale.**

(30) Priorité : **21.03.86 FR 8604109**

(43) Date de publication de la demande :
**30.09.87 Bulletin 87/40**

(45) Mention de la délivrance du brevet :
**06.11.91 Bulletin 91/45**

(84) Etats contractants désignés :
**AT BE CH DE ES FR GB GR IT LI LU NL SE**

(56) Documents cités :
**EP-A- 0 169 974**
**FR-A- 2 416 004**
**FR-A- 2 519 545**
**US-A- 4 524 467**

(73) Titulaire : **Perrin, Max**
**Gouville**
**F-21160 Marsannay La Côte (FR)**

(72) Inventeur : **Perrin, Max**
**Gouville**
**F-21160 Marsannay La Côte (FR)**

(74) Mandataire : **CABINET BONNET-THIRION**
**95 Boulevard Beaumarchais**
**F-75003 Paris (FR)**

## Description

La présente invention concerne d'une manière générale les prothèses cotyloïdiennes, et elle vise plus particulièrement, mais non nécessairement exclusivement, les prothèses coxo-fémorales, c'est-à-dire les prothèses de hanche.

Plus particulièrement encore, la présente invention concerne la partie d'une telle prothèse, ou cotyle, destinée à fournir la surface articulaire concave de l'articulation à reconstituer.

S'agissant d'une prothèse de hanche, un tel cotyle est à ancrer dans la cavité osseuse de l'os iliaque.

A ce jour, deux modes d'ancrage sont envisageables, l'un avec ciment, l'autre sans ciment, et ils ont chacun leurs avantages et leurs inconvénients.

L'ancrage sans ciment, s'il permet normalement d'espérer une liaison définitive, impose usuellement, de prime abord, une mobilisation prudente de l'articulation jusqu'à ce que la repousse osseuse soit suffisante pour assurer une consolidation satisfaisante.

L'ancrage avec ciment, qui reste en pratique à ce jour le plus utilisé, conduit par contre à une tenue immédiate très satisfaisante autorisant rapidement la déambulation du patient et sa rééducation.

Mais le ciment, qui est en fait une résine acrylique, en pratique du métacrylate de méthyle, à faire polymériser in situ, et qui joue le rôle d'un matelas de remplissage entre le cotyle et la cavité osseuse, a pour inconvénient d'être, à terme, l'objet d'un certain vieillissement, et, dans certains cas au moins, c'est à un tel vieillissement que l'on peut attribuer les descellements qu'il est parfois possible d'observer.

On peut penser que ce vieillissement est notamment dû à une certaine sensibilité à la fatigue.

En effet, du fait que, à ce jour, le ciment ainsi mis en oeuvre est seul à transmettre à la cavité osseuse les efforts en provenance de la tête d'articulation correspondante, il est soumis à des contraintes non négligeables, qui en accroissent progressivement le durcissement, alors même que, dès l'origine, il est plus dur que l'os.

Il y a ainsi progressivement, du point de vue de la dureté, une détérioration des conditions de compatibilité de ce ciment avec l'os, ce dernier étant au contraire toujours capable, par micro-déformations, d'une certaine élasticité, et l'ensemble s'écarte donc progressivement de l'isostatisme qui serait préférable.

En outre, par elle-même, la mise en oeuvre de ciment n'est pas sans soulever des difficultés.

Tout d'abord, sa mise en place dans la cavité osseuse se fait nécessairement au doigt, et il en résulte que, mal contrôlée, sa répartition dans celle-ci demeure aléatoire, même s'il est prévu d'en mettre en excès, le cotyle prothétique à ancrer présentant usuellement, sur la partie convexe de sa surface extérieure, et suivant des méridiens de celle-ci, des rainures propres à en récupérer le surplus et à le canaliser vers l'extérieur sous l'effet de la pression de mise en place qui lui est appliquée.

En outre, le praticien doit à ce jour maintenir à la main le cotyle pendant tout le temps nécessaire à la prise du ciment, ce qui le mobilise inutilement pendant un temps non négligeable et prolonge d'autant l'intervention, sans que soient pour autant écartés les risques d'éventuels mouvements intempestifs du cotyle pendant une telle prise.

La présente invention a d'une manière générale pour objet une disposition permettant d'éviter ces inconvénients et conduisant en outre à d'autres avantages.

De manière plus précise, elle a pour objet un cotyle pour prothèse cotyloïdienne, notamment pour prothèse coxo-fémorale, caractérisé d'une manière générale en ce qu'il présente, en creux, sur la partie convexe de sa surface extérieure, au moins trois rainures, et en ce que il lui est associé un nombre égal de chevilles à implanter dans la cavité osseuse à équiper, de telle manière que sa mise en place dans cette cavité osseuse se fasse sur ces chevilles suivant un montage du type montage à baïonnette.

Autrement dit, suivant l'invention, il est d'abord procédé à la mise en place, dans la cavité osseuse à équiper, d'un certain nombre de chevilles, en pratique trois, en tirant parti pour ce faire de la partie en "sourcil" de cette cavité osseuse présentant, autour de son "arrière fond", une épaisseur d'os cortical, dur, plus grande, et, après bourrage éventuel, et préférentiel, de la cavité osseuse par du ciment, il est ensuite rapporté, sur ces chevilles, qui font chacune saillie sur son fond, le cotyle dont cette cavité osseuse doit être équipée.

Grâce à une telle disposition, et à la manière des prothèses à ancrage sans ciment, les chevilles ainsi mises en oeuvre assurent avantageusement une transmission directe des contraintes du cotyle à l'os de la cavité osseuse concernée, ce qui ménage d'autant le ciment présent par ailleurs, lorsque, comme cela est le cas préférentiel, parce qu'il sert alors d'adaptateur entre le cotyle et la cavité osseuse, en bouchant les irrégularités de surface de celle-ci, un tel ciment est effectivement lui aussi mis en oeuvre.

Ainsi soustrait aux contraintes mécaniques, ce ciment vieillit dans de meilleures conditions, et ses risques de descellement s'en trouvent avantageusement minimisés.

En outre, les conséquences d'un tel éventuel descellement du ciment se trouvent elles-mêmes minimisées, la pérennité de l'ancrage du cotyle pouvant de toute façon être assurée par les seules chevilles avec lesquelles il est en prise.

Ainsi, grâce à la disposition suivant l'invention, suivant laquelle, indépendamment d'un éventuel matelas de ciment, le cotyle est en quelque sorte suspendu, à la manière d'un tripode, à des chevilles, en se bloquant de lui-même sur celles-ci, il est assuré un avantageux compromis entre les prothèses à ancrage avec ciment et les prothèses à ancrage sans ciment.

Mais la mise en oeuvre de telles chevilles présente d'autres avantages.

Tout d'abord, elles garantissent d'un bon centrage du cotyle dans la cavité osseuse.

En outre, en fournissant un appui au cotyle lors de son vissage sur elles, elles conduisent à une mise sous pression, par ce cotyle, du matelas de ciment éventuellement préalablement mis en place dans la cavité osseuse, ce qui est favorable à sa bonne répartition dans celle-ci, en l'obligeant à envahir au mieux toutes ses irrégularités de surface, aussi bien d'ailleurs que les rainures, saignées ou gorges prévues par ailleurs sur le cotyle pour son ancrage.

De plus, plus aucun déplacement intempestif du cotyle n'est à craindre pendant la prise du ciment.

En outre, affranchi de la nécessité actuelle du maintien du cotyle pendant cette prise, le praticien peut avantageusement procéder pendant celle-ci à une autre partie du travail qu'il a à effectuer, et par exemple à la préparation et à la mise en place de la prothèse fémorale, ce qui réduit avantageusement d'autant la durée d'ensemble de son intervention et ménage donc en conséquence le patient.

Par ailleurs, grâce aux chevilles mises en oeuvre suivant l'invention, il est possible d'envisager le traitement de cas actuellement difficiles à traiter, comme celui des coxarthroses protusives.

En effet, le cotyle prothétique suivant l'invention étant en quelque sorte, comme indiqué ci-dessus, suspendu à de telles chevilles, le fond de la cavité osseuse à equiper, qui est éventuellement fragilisé, est avantageusement soustrait à toute pression de sa part.

Par ailleurs, et cela peut être particulièrement intéressant pour les praticiens manquant encore d'expérience, ces chevilles permettent avantageusement de procéder à un essai préalable, in situ, du cotyle qu'ils s'apprêtent à poser, sans scellement de celui-ci.

Il leur est ainsi possible de choisir au mieux ce cotyle, et, par une réduction temporaire de l'ensemble, de choisir également au mieux la longueur de la tête de fémur correspondante eu égard à la tension musculaire du patient.

Rien n'interdit d'ailleurs, après un tel essai, de modifier si nécessaire la place du cotyle, en modifiant l'implantation des chevilles sur lesquelles il doit être vissé.

Enfin, par un réglage de leur position, et donc de la saillie qu'elles forment sur le fond de la cavité osseuse, ces chevilles permettent avantageusement d'ajuster au mieux à une telle cavité osseuse un cotyle de dimension déterminée, ce qui est favorable à une minimisation du nombre de cotyles à prévoir pour convenir à l'ensemble des patients à traiter, et ce qui est donc favorable à une standardisation économique de ces cotyles.

Certes, dans la demande de brevet français publiée sous le No FR-A-2.416.004, il est fait état d'un montage à baïonnette ; mais ce montage à baïonnette intervient entre une cupule externe et une cupule interne, et non, suivant l'invention, entre l'ensemble et l'excavation osseuse à équiper.

Les cotyles en cause sont donc de natures différentes et leurs mises en place respectives se font également de manières différentes.

Les caractéristiques et avantages de l'invention ressortiront d'ailleurs de la description qui va suivre, à titre d'exemple, en référence aux dessins schématiques annexés sur lesquels :

la figure 1 est une vue en perspective d'un cotyle suivant l'invention et de l'os dans une cavité duquel il doit être rapporté ;

la figure 2 est, à échelle supérieure, une vue latérale de bout de ce cotyle, suivant la flèche II de la figure 1 ;

la figure 3 en est une vue en élévation, suivant la flèche III de la figure 2 ;

la figure 4 en est une vue partielle en coupe axiale, suivant la ligne IV-IV de la figure 2 ;

la figure 5 est une vue en élévation d'une des chevilles propres à la mise en place du cotyle suivant l'invention ;

la figure 6 est, suivant la ligne VI-VI de la figure 5, une vue en coupe transversale de cette cheville, avec, à distance, la goupille qui lui est associée ;

la figure 7 est une vue analogue à celle de la figure 6, après mise en place de cette goupille ;

les figures 8A, 8B, 8C sont, à la manière de la figure 4, des vues partielles en coupe axiale du cotyle suivant l'invention illustrant diverses phases de mise en place de celui-ci dans la cavité osseuse à équiper ;

la figure 9 est une vue analogue à celle de la figure 8C, pour un autre réglage en position des chevilles concernées ;

la figure 10 est une vue latérale de bout qui, analogue à celle de la figure 2, se rapporte à une variante de réalisation du cotyle suivant l'invention ;

la figure 11 est, à la manière de la figure 4, une vue partielle en coupe axiale de cette variante de réalisation, suivant la ligne XI-XI de la figure 10 ;

la figure 12 illustre à la manière de la figure 8C, la mise en place du cotyle suivant l'invention dans cette variante de réalisation.

Ces figures illustrent, à titre d'exemple, l'application de l'invention à une prothèse coxo-fémorale.

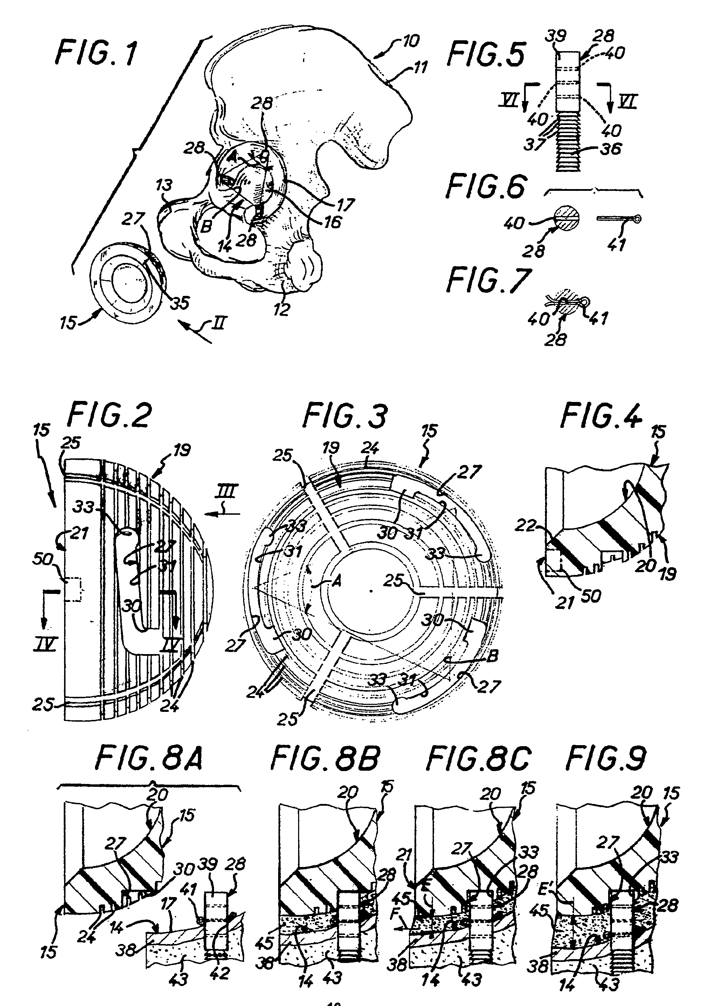

A la figure 1, on reconnaît, sous la référence générale 10, l'os de hanche, ou os iliaque, à équiper.

Il s'agit, en l'espèce, d'un os gauche.

Formé, ainsi qu'on le sait, de trois os, l'ilion 11, l'ischion 12, et le pubis 13, il présente, à la jonction de ceux-ci, une cavité osseuse 14 dans laquelle doit être implanté le cotyle 15 de la prothèse à mettre en place.

Autour d'une partie centrale 16, ou "arrière fond", où l'épaisseur de l'os cortical, dur, est relativement faible, cette cavité osseuse 14 présente, en croissant, une partie 17, dite "sourcil", où cette épaisseur est plus grande.

Globalement, et de manière connue en soi, le cotyle 15 se présente sous la forme d'une pièce de révolution dont la surface extérieure comporte une partie convexe 19, par laquelle il doit être engagé dans la cavité osseuse 14 à équiper, une partie concave 20, par laquelle il est adapté à coopérer avec la tête de fémur correspondante, et, de l'une à l'autre de ces deux parties, sensiblement perpendiculairement à l'axe de l'ensemble, une face frontale 21.

Dans les formes de réalisation représentées, la partie convexe 19 de cette surface extérieure est sensiblement hémisphérique, à l'image de la forme naturelle de la cavité osseuse 14 à équiper.

Mais il s'agit là d'une disposition qui, sans incidence sur l'objet même de la présente invention, ne doit en rien être considérée comme limitative de celle-ci.

Au contraire, le profil transversal de la partie convexe 19 de cette surface extérieure peut être différent de celui d'un arc de cercle.

Il peut par exemple s'agir aussi bien d'un arc de parabole, d'un arc d'ellipse, ou d'une courbe quelconque, voire même d'une droite, la partie convexe 19 de la surface extérieure du cotyle 15 suivant l'invention étant alors globalement tronconique.

Quoi qu'il soit, et de manière connue en soi, la partie concave 20 de la surface extérieure du cotyle 15 suivant l'invention forme, toujours, elle, une calotte sphérique, et celle-ci s'étend, ou non, sur plus de 180°, si elle est rétentive.

Dans les formes de réalisation représentées, elle se raccorde par une portion tronconique 22 à la face frontale 21 à son débouché.

De manière également connue en soi, la partie convexe 19 de la surface extérieure du cotyle 15 suivant l'invention est affectée, en creux, d'une part, d'une pluralité de rainures 24, qui, établies suivant des parallèles, sont échelonnées les unes par rapport aux autres le long de son axe, et, d'autre part, d'une pluralité de rainures 25 qui, recoupant les précédentes, s'étendent elles, en étoile, suivant des méridiens.

Dans les formes de réalisation représentées, les rainures 24 sont relativement étroites et profondes, et les rainures 25, qui sont plus larges, et qui sont en nombre plus limité, débouchent à la surface de la face frontale 21.

Suivant l'invention, le cotyle 15 présente, en outre, en creux, sur la partie convexe 19 de sa surface extérieure, au moins trois rainures 27 suivant un montage du type montage à baïonnette, et, tel que décrit plus en détail ultérieurement, il lui est associé un nombre égal de chevilles 28 à implanter dans la cavité osseuse 14 à équiper, de telle manière que sa mise en place dans cette cavité osseuse 14 se fasse sur ces chevilles 28 suivant un montage du type montage à baïonnette.

Dans les formes de réalisation représentées, trois rainures 27 sont ainsi prévues, et, prises dans leur ensemble, et tel que schématisé en traits interrompus, en plan, à la figure 3, elles sont globalement disposées aux sommets d'un triangle isocèle dont l'angle A opposé à la base B est inférieur à 60°.

Vues latéralement, figure 2, elles s'étendent sensiblement toutes à un même niveau, qui est en pratique globalement aux deux tiers de la hauteur du cotyle 15 à compter de sa zone apicale, ou, autrement dit, au tiers de sa hauteur à compter de sa face frontale 21.

Chacune des rainures 27 ainsi prévues suivant l'invention comporte un tronçon d'entrée 30, qui, à partir de son débouché sur la partie convexe 19 de la surface extérieure du cotyle 15, s'étend sensiblement axialement en direction de la face frontale 21 de celui-ci, et qui, au moins audit débouché, est de préférence évasé, et un tronçon de partie courante 31, qui, à partir dudit tronçon d'entrée 30, s'étend globalement circulairement tout en étant légèrement en oblique par rapport à ladite face frontale 21, ledit tronçon de partie courante 31 se rapprochant de cette face frontale 21 au fur et à mesure qu'il s'éloigne du tronçon d'entrée 30 auquel il fait ainsi suite.

En pratique, et tel que représenté, le tronçon de partie courante 31 de chaque rainure 27 est sensiblement rectiligne, et sa pente est faible, en étant inférieure à 0,1 par exemple.

Dans la forme de réalisation plus particulièrement représentée sur les figures 1 à 9, ce tronçon de partie courante 31 de chaque rainure 27 présente, en creux, à son extrémité opposée au tronçon d'entrée 30 auquel il fait suite, qui est borgne, sur celui de ses flancs qui est axialement tourné en direction de la face frontale 21, un épanouissement 33.

Dans la forme de réalisation représentée, cet épanouissement 33, qui est en pratique peu accentué, en étant par exemple de l'ordre de 0,1 à 0,2 mm, est globalement arrondi.

Quoi qu'il en soit, il s'étend axialement dans le même sens que le tronçon d'entrée 30, et donc en direction opposée à la face frontale 21 à partir du tronçon de partie courante 31.

En outre, dans cette forme de réalisation, les génératrices des flancs du tronçon de partie courante 31 des rainures 27 que présente ainsi le cotyle 15 suivant l'invention sont sensiblement perpendiculaires à son axe.

En pratique, elles sont ainsi, dans ce cas, sensiblement parallèles à sa face frontale 21.

De préférence, et tel que visible à la figure 1, le cotyle 15 suivant l'invention comporte, sur sa face frontale 21, au moins un repère 35 dont l'implantation sur cette face frontale 21 est en relation avec l'implantation de ses rainures 27 sur la partie convexe 19 de sa surface extérieure.

Il peut s'agir par exemple d'une simple saignée radiale affectant en creux une telle face frontale 21.

De manière connue en soi, le cotyle 15 suivant l'invention peut avantageusment être réalisé en matière synthétique, et par exemple en polyéthylène.

Bien entendu, d'autres matériaux sont cependant envisageables, et, par exemple, des matériaux métalliques et des céramiques.

Mais, de préférence, le matériau constitutif des chevilles 28 associées à ce cotyle 15 est de même nature que celui de ce dernier et il lui est de préférence identique.

Dans les formes de réalisation représentées, le fût 36 des chevilles 28 présente, transversalement, sur une partie au moins de sa hauteur, et en pratique sur la totalité de celle-ci, des saillies d'ancrage 37, tels que ailettes ou barbillons.

La tête 39 des chevilles 28, par contre, est lisse.

Cette tête 39, au moins, a, transversalement, un contour circulaire.

Dans les formes de réalisation représentées, il en est de même pour le fût 36 qu'elle surmonte.

Dans la forme de réalisation plus particulièrement représentée sur les figures 1 à 9, chacune des chevilles 28 ainsi constituées présente, transversalement, au moins un perçage 40, pour la mise en place d'une goupille d'arrêt 41.

En pratique, chacune des chevilles 28 présente ainsi, transversalement, échelonnés à distance l'un de l'autre sur sa longueur, parallèlement les uns aux autres, une pluralité de perçages 40.

A titre d'exemple, ces perçages 40 sont au nombre de trois dans la forme de réalisation représentée.

Ils affectent tous la tête 39, lisse, de ces chevilles 28.

Pour la mise en oeuvre du cotyle 15 suivant l'invention il est procédé de la manière suivante, après les interventions usuelles sur la cavité osseuse à équiper.

Tout d'abord, il est procédé au forage, dans la partie 17, en croissant, de cette cavité osseuse 14, qui est celle où l'épaisseur de l'os cortical, dur, est la plus importante, de trois trous 42 propres chacun à recevoir une cheville 28.

Bien entendu, de tels trous ne concernent pas seulement cet os cortical, qui est désigné par la référence 38 sur les figures 8, mais ils s'étendent également dans le tissu spongieux 43 sous-jacent à celui-ci.

Bien entendu, également, l'implantation des trous 42 ainsi pratiqués dans la cavité osseuse 14 est faite à l'image de celle des rainures 27 du cotyle 15 à y mettre en place.

Autrement dit, et tel que schématisé en traits interrompus à la figure 1, elle est faite suivant les sommets d'un triangle isocèle dont l'angle A opposé à la base B est inférieur à 60°.

En pratique, le sommet de ce triangle correspondant à cet angle A intéresse l'ilion 11, tandis que les sommets disposés aux extrémités de la base B intéressent chacun respectivement l'ischion 12 et le pubis 13.

C'est d'ailleurs, pour une meilleure adaptation à la morphologie de l'os iliaque 10 ici concerné, que, dans les formes de réalisation et de mise en oeuvre représentées, le triangle aux sommets desquels se trouvent les rainures 27 du cotyle 15 suivant l'invention n'est pas un triangle équilatéral.

Mais il va de soi que d'autres positions peuvent aussi être envisagées.

pour faciliter l'implantation des trous 42, un gabarit peut avantageusement être utilisé.

Dans le cas de la forme de réalisation décrite cidessus en référence aux figures 1 à 9, ces trous 42 sont préférentiellement forés sensiblement perpendiculairement à l'axe de la cavité osseuse 14, et sensiblement dans un même plan.

Mais, d'autres orientations peuvent être envisageables avec, le cas échéant, une adaptation correspondante pour le cotyle 15.

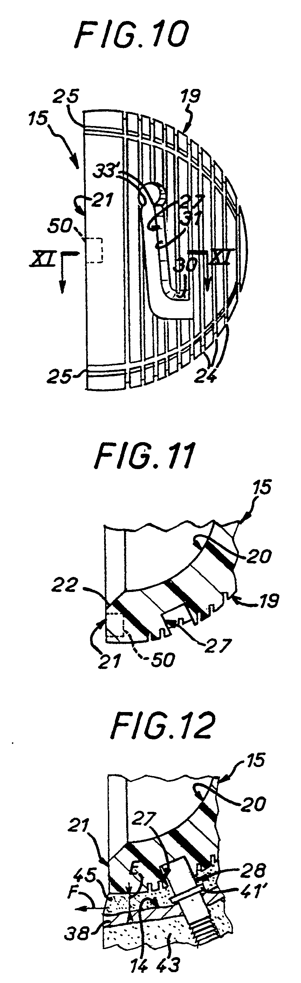

C'est le cas, notamment, pour la forme de réalisation, qui, représentée sur les figures 10 à 12, sera décrite ultérieurement.

Quoi qu'il en soit, pour forer les trous 42, il doit normalement être utilisé des renvois d'angle.

Il est procédé ensuite à l'enfoncement de chevilles 28 dans ces trous 42.

Suivant les dimensions relatives du cotyle 15 par rapport à celles de la cavité osseuse 14, cet enfoncement est plus ou moins accentué, de manière à ajuster à la valeur désirée l'intervalle E, à combler par du ciment 45, entre ce cotyle 15 et cette cavité osseuse 14.

Sur les figures 8B et 8C, l'intervalle E a été volontairement exagéré.

Il en est de même sur la figure 9, où il s'agit d'un intervalle E' encore plus grand, la cavité osseuse 14 correspondante étant supposée plus importante.

En pratique, cet intervalle E ou E' est beaucoup plus réduit.

En pratique, également, dans le cas de la forme de réalisation représentée sur les figures 1 à 9, le réglage en position d'une cheville 28 se fait à l'aide d'une goupille 41, par engagement de cette goupille 41 dans celui des perçages 40 d'une telle cheville 28 correspondant à la position recherchée pour celle-ci.

Une fois en place dans ce perçage 40, la goupille 41 fait en effet office de garde, en venant buter contre le fond de la cavité osseuse 14 lors de l'enfoncement de la cheville 28 qu'elle équipe dans le trou 42 correspondant de celle-ci.

Bien entendu, le diamètre des chevilles 28 ainsi mises en oeuvre est choisi de manière à être bien adapté à celui des trous 42 pratiqués.

Ainsi qu'il est aisé de le comprendre, les saillies 37 que présentent préférentiellement ces chevilles 28 facilitent cependant aussi bien leur adaptation convenable à ces trous 42 que leur ancrage souhaitable dans ceux-ci.

Mais, préférentiellement, et tel que représenté, les chevilles 28 doivent s'appuyer par leur tête 39 lisse dans l'os cortical 38 traversé, leur fût ailetté 36 étant plus particulièrement destiné, lui, au tissu spongieux 43 sous-jacent.

C'est la raison pour laquelle les perçages 40 de ces chevilles 28 affectent tous la tête lisse 39 de celles-ci, avec, en sus, une garde correspondant à l'épaisseur de l'os cortical en question.

Toutes les chevilles 28 à mettre en oeuvre étant ainsi en place, il est procédé ensuite, si désiré, et, en pratique, de manière préférentielle, au tapissage du fond de la cavité osseuse 14 par du ciment 45.

Le cotyle 15 est alors présenté au droit de la cavité osseuse 14, figure 8A, avec le tronçon d'entrée 30 de chacune de ses rainures 27 en regard de la cheville 28 correspondante ainsi en saillie sur le fond de celle-ci.

Ainsi qu'il est aisé de le comprendre, cette présentation du cotyle 15 en concordance avec les chevilles 28 se trouve facilitée par le repère 35 prévu à cet effet sur sa face frontale 21, et son engagement sur ces chevilles 28 se trouve lui-même facilité par le caractère évasé du débouché, au moins, du tronçon d'entrée 30 de ses rainures 27.

L'engagement du cotyle 15 sur les chevilles 28 étant poursuivi, figure 8B, il vient en butée contre ces chevilles 28 par celui des flancs du tronçon de partie courante 31 de ses rainures 27 qui est le plus proche de sa face frontale 21.

Il suffit, ensuite, d'imprimer au cotyle 15 un mouvement de vissage, c'est-à-dire un mouvement de rotation sur lui-même autour de son axe.

Au cours de ce mouvement de vissage, le cotyle 15 vient en prise avec les chevilles 28 par le tronçon de partie courante 31 de ses rainures 27, et, du fait de l'obliquité de celui-ci, il pénètre plus avant dans la cavité osseuse 14.

Il en résulte une mise sous pression du ciment 45, en sorte que ce ciment 45 vient s'insérer aussi bien dans tous les interstices ou enfractuosités du fond de la cavité osseuse 14 que dans les rainures parallèles 24 ou méridiennes 25 du cotyle 15, cependant que sa partie en excès est expulsée vers l'extérieur, suivant la flèche F de la figure 8C, à la faveur notamment de ces dernières.

Au terme de son vissage, et sous les effets d'une réaction élastique du ciment 45, dont il résulte pour lui une certaine force d'expulsion, le cotyle 15, dans la forme de réalisation représentée sur les figures 1 à 9, vient en prise avec les chevilles 28 par les épanouissements d'extrémité 33 de ses rainures 27, figure 8C, ce qui assure l'irréversibilité de son vissage.

En outre, il résulte de l'engagement des chevilles 28 dans ces épanouissements 33 l'émission d'un bruit caractéristique signalant au praticien que le vissage du cotyle 15 est achevé.

Bien entendu, lors du positionnement des chevilles 28 dans la cavité osseuse 14, il est tenu compte de l'enfoncement dans celle-ci dont est l'objet le cotyle 15 lors de son vissage sur ces chevilles 28 du fait de l'obliquité du tronçon de partie courante 31 de ses rainures 27.

Ainsi qu'on l'aura compris, le vissage du cotyle 15 sur les chevilles 28 se pratique avantageusement à l'aide d'un outil approprié, manche de vissage par exemple, des moyens d'emboîtement complémentaires étant prévus à cet effet entre un tel cotyle 15 et un tel outil.

Par exemple, et tel que schématisé en traits interrompus sur les figures 2 et 4, il peut être prévu à cet effet de place en place, en creux sur la face frontale 21 du cotyle 15, des cuvettes 50.

A l'inverse, de telles cuvettes 50 peuvent être remplacées par des saignées, rainures ou autres évidements ou orifices, voire même par des ergots en saillie.

Dans la variante de réalisation représentée sur les figures 10 à 12, les génératrices des flancs du tronçon de partie courante 31 des rainures 27 du cotyle 15 suivant l'invention sont obliques par rapport à son axe.

En pratique, elles s'étendent sensiblement perpendiculairement à sa surface extérieure.

Dans le cas où, tel que représenté, celle-ci est sensiblement hémisphérique, elles s'étendent donc sensiblement radialement, ou, plus exactement, sensiblement parallèlement à la direction radiale de cette surface extérieure passant par une ligne médiane entre les flancs concernés.

En outre, dans cette variante de réalisation, au lieu d'un épanouissement 33, le tronçon de partie courante 31 de l'une au moins des rainures 27, et, en pratique, de chacune de celles-ci, présente, à proximité de son extrémité opposée au tronçon d'entrée 30 correspondant, en saillie sur l'un au moins de ses flancs, un bossage 33'.

Dans la forme de réalisation représentée, il y a ainsi un bossage 33' sur l'un et l'autre des flancs concernés, et ces bossages 33', que doit franchir à force la cheville 28 correspondante lors du vissage de l'ensemble, sont peu accentués, en présentant, en rebroussement, une arête sommitale relativement aiguë.

Enfin, dans cette variante de réalisation, les chevilles 28 présentent chacune d'un seul tenant une garde d'arrêt 41'.

Par exemple, et tel que représenté, celle-ci se présente sous la forme d'une collerette faisant annulairement saillie à la surface d'une telle cheville.

De préférence, et tel qu'également représenté, il subsiste, sous cette collerette, pour les raisons déjà exposées, un tronçon lisse.

La mise en place de cette variante de réalisation se fait comme précédemment.

La présente invention ne se limite d'ailleurs pas aux formes de réalisation décrites et représentées, mais englobe toute variante d'exécution et/ou de combinaison de leurs divers éléments.

En particulier, bien que la mise en oeuvre de ciment soit préférentielle, on ne sortirait pas du cadre général de l'invention en procédant sans ciment.

Par exemple, pour assurer dans ce cas l'irréversibilité du vissage du cotyle suivant l'invention au terme de sa mise en place, il peut être tiré profit de l'élasticité propre du fond de la cavité osseuse concernée, en choisissant en conséquence les dimensions de ce cotyle.

En variante, il peut être mis en place, au fond de cette cavité osseuse, un quelconque tampon ou autre moyen élastique.

En outre, pour le montage à baïonnette à assurer, un nombre de rainures supérieur à trois est envisageable et/ou ces rainures ne s'étendent pas toutes nécessairement à un même niveau.

Corollairement, pour faciliter leur engagement sur les chevilles mises en oeuvre, le tronçon d'entrée de ces rainures peut être élargi, aux lieu et place d'un évasement du seul débouché de ce tronçon d'entrée ou en coopération avec un tel évasement.

De plus, au lieu d'être enfoncées dans la cavité osseuse à équiper, les chevilles correspondantes peuvent tout aussi bien y être vissées.

Corollairement, le domaine d'application de l'invention n'est pas lui non plus limité à celui des seules prothèses de hanche, mais s'étend au contraire tout aussi bien à celui de toutes les autres prothèses articulaires.

Dans le cas de la forme de réalisation représentée sur les figures 1 à 9, un réglage en position des chevilles correspondantes dans la cavité osseuse permet, avec une large tolérance, de disposer un même cotyle dans des cavités osseuses de dimensions plus ou moins différentes ; il suffit de choisir en conséquence le perçage de ces chevilles dans lequel doit être engagée la goupille leur servant de garde d'arrêt.

Par ailleurs, un démontage est toujours possible : il suffit de perforer le cotyle jusqu'aux têtes des chevilles qui le retiennent et de l'extraire ensuite à l'aide d'un davier.

Enfin, le cotyle suivant l'invention peut aussi bien être mis en oeuvre avec un "fond de cotyle", c'est-à-dire une armure, en pratique métallique, préalablement rapportée au fond de la cavité osseuse à équiper.

Il suffit en effet de munir cette armure de pointes propres à y assujettir les chevilles, si celles-ci se trouvent ainsi implantées dans la cavité osseuse par son intermédiaire

**Revendications**

1. Cotyle pour prothèse cotyloïdienne, notamment pour prothèse coxo-fémorale, caractérisé en ce qu'il présente, en creux, sur la partie convexe (19) de sa surface extérieure, au moins trois rainures (27), et en ce qu'il lui est associé un nombre égal de chevilles (28) à implanter dans la cavité osseuse (14) à équiper, de telle manière que sa mise en place dans cette cavité osseuse (14) se fasse sur ces chevilles (28) suivant un montage du type montage à baïonnette.

2. Cotyle suivant la revendication 1, caractérisé en ce que chacune de ses rainures (27) comporte un tronçon d'entrée (30), qui, à partir de son débouché sur la partie convexe (19) de la surface extérieure du cotyle, s'étend sensiblement axialement en direction de la face frontale (21) de celui-ci, et un tronçon de partie courante (31), qui, à partir dudit tronçon d'entrée (30), s'étend globalement circulairement tout en étant légèrement en oblique par rapport à ladite face frontale (21), ledit tronçon de partie courante (31) se rapprochant de cette face frontale (21) au fur et à mesure qu'il s'éloigne du tronçon d'entrée (30) auquel il fait suite.

3. Cotyle suivant la revendication 2, caractérisé en ce que le tronçon de partie courante (31) d'une rainure (27) est sensiblement rectiligne.

4. Cotyle suivant l'une quelconque des revendications 2, 3, caractérisé en ce que, à son extrémité opposée au tronçon d'entrée (30), le tronçon de partie courante (31) d'une rainure (27) présente, en creux, sur celui de ses flancs qui est axialement tourné en direction de la face frontale (21) du cotyle, un épanouissement (33).

5. Cotyle suivant l'une quelconque des revendications 2, 3, caractérisé en ce que, à proximité de son extrémité opposée au tronçon d'entrée (30), le tronçon de partie courante (31) d'une rainure (27) présente, en saillie, sur l'un au moins de ses flancs, un bossage (33').

6. Cotyle suivant l'une quelconque des revendications 1 à 5, caractérisé en ce que les génératrices des flancs du tronçon de partie courante (31) de ses rainures (27) sont sensiblement perpendiculaires à son axe.

7. Cotyle suivant l'une quelconque des revendications 1 à 5, caractérisé en ce que les génératrices des flancs du tronçon de partie courante (31) de ses rainures (27) sont obliques par rapport à son axe.

8. Cotyle suivant la revendication 7, caractérisé en ce que les génératrices des flancs du tronçon de partie courante (31) de ses rainures (27) s'étendent sensiblement perpendiculairement à sa surface extérieure.

9. Cotyle suivant l'une quelconque des revendications 1 à 8, caractérisé en ce qu'il comporte trois rainures (27) disposées aus sommets d'un triangle isocèle dont l'angle (A) opposé à la base (B) est inférieur à 60°.

10. Cotyle suivant l'une quelconque des revendications 1 à 9, caractérisé en ce qu'il comporte sur sa face frontale (21) au moins un repère (35) dont l'implantation sur cette face frontale (21) est en relation avec l'implantation de ses rainures (27) sur la partie convexe (19) de sa surface extérieure.

11. Cotyle suivant l'une quelconque des revendications 1 à 10, caractérisé en ce que chacune des chevilles (28) qui lui sont associées présente transversalement au moins un perçage (40), pour la mise en place d'une goupille d'arrêt (41).

12. Cotyle suivant la revendication 11, caractérisé en ce que chacune des chevilles (28) présente transversalement, échelonnés à distance l'un de l'autre sur sa longueur, une pluralité de perçages (40).

13. Cotyle suivant l'une quelconque des revendications 1 à 10, caractérisé en ce que chacune des chevilles (28) qui lui sont associées présente d'un seul tenant une garde d'arrêt (41').

14. Cotyle suivant l'une quelconque des revendications 1 à 13, caractérisé en ce que chacune des chevilles (28) qui lui sont associées a une tête lisse (39) et un fût (36) présentant transversalement des saillies d'ancrage (37), tels que ailettes ou barbillons.

15. Cotyle suivant l'une quelconque des revendications 1 à 14, caractérisé en ce que la tête (39) au moins des chevilles (28) qui lui sont associées a transversalement un contour circulaire.

16. Cotyle suivant l'une quelconque des revendications 1 à 15, caractérisé en ce que le matériau des chevilles (28) qui lui sont associées est de même nature que son propre matériau constitutif et est de préférence identique à celui-ci.

**Patentansprüche**

1. Gelenkpfanne für eine Gelenkpfannenprothese, besonders für eine Hüftgelenkprothese, dadurch gekennzeichnet, daß sie auf dem vorgewölbten Teil (19) ihrer Außenfläche vertieft mindestens drei Nuten (27) aufweist und daß ihr eine gleiche Zahl von in die mit ihr zu versehende Knochenhöhlung (14) einzusetzenden Stiften (28) in der Weise zugeordnet ist, daß das Einsetzen der Gelenkpfanne in diese Knochenhöhlung (14) auf diese Stifte (28) gemäß einer Montage von der Art einer Bajonettmontage erfolgt.

2. Gelenkpfanne nach Anspruch 1, dadurch gekennzeichnet, daß jede dieser Nuten (27) einen Eingangsabschnitt (30) aufweist, der sich ausgehend von seiner Mündung auf dem vorgewölbten Teil (19) der Außenfläche der Gelenkpfanne im wesentlichen axial in Richtung der Stirnfläche (23) der Gelenkpfanne erstreckt, und einen Streckenabschnitt (31) aufweist, der sich ausgehend vom Eingangsabschnitt (30) im ganzen kreisförmig, dabei jedoch etwas schräg bezüglich der Stirnfläche (21) erstreckt, wobei sich dieser Streckenabschnitt (31) dieser Stirnfläche (21) in dem Maße annähert, wie er sich von dem Eingangsabschnitt (30) entfernt, auf den er folgt.

3. Gelenkpfanne nach Anspruch 2, dadurch gekennzeichnet, daß der Streckenabschnitt (31) einer Nut (27) im wesentlichen geradlinig ist.

4. Gelenkpfanne nach einem der Ansprüche 2 und 3, dadurch gekennzeichnet, daß der Streckenabschnitt (31) einer Nut (27) an seinem dem Eingangsabschnitt (30) entgegengesetzen Ende an derjenigen seiner Flanken, die axial der Stirnseite (21) der Gelenkpfanne zugewandt ist, vertieft eine Ausbuchtung (33) aufweist.

5. Gelenkpfanne nach einem der Ansprüche 2 und 3, dadurch gekennzeichnet, daß der Streckenabschnitt (31) einer Nut (27) in der Nähe seines dem Eingangsabschnitt (30) entgegengesetzten Endes auf mindestens einer seiner Flanken einen vorspringenden Buckel (33') aufweist.

6. Gelenkpfanne nach einem der Ansprüche 1 bis 5, dadurch gekennzeichnet, daß die Mantellinien der Flanken des Streckenabschnitts (31) ihrer Nuten (27) im wesentlichen senkrecht zu ihrer Achse verlaufen.

7. Gelenkpfanne nach einem der Ansprüche 1 bis 5, dadurch gekennzeichnet, daß die Mantellinien der Flanken des Streckenabschnitts (31) ihrer Nuten (27) schräg zu ihrer Achse verlaufen.

8. Gelenkpfanne nach Anspruch 7, dadurch gekennzeichnet, daß die Mantellinien der Flanken des Streckenabschnitts (31) ihrer Nuten (27) im wesentlichen senkrecht zu ihrer Außenfläche verlaufen.

9. Gelenkpfanne nach einem der Ansprüche 1 bis 8, dadurch gekennzeichnet, daß sie drei Nuten (27) aufweist, die an den Spitzen eines gleichschenkeligen Dreiecks angeordnet sind, dessen seiner Basis (B) gegenüberliegender Winkel (A) kleiner als 60° ist.

10. Gelenkpfanne nach einem der Ansprüche 1 bis 9, dadurch gekennzeichnet, daß sie auf ihrer Stirnfläche (21) mindestens eine Markierung (35) trägt, deren Anordnung auf dieser Stirnfläche (21) in einer Beziehung zur Anordnung ihrer Nuten (27) auf dem vorgewölbten Teil (19) ihrer Außenfläche steht.

11. Gelenkpfanne nach einem der Ansprüche 1 bis 10, dadurch gekennzeichnet, daß jeder der Stifte (28), die ihr zugeordnet sind, mindestens eine Querbohrung (40) zum Einsetzen eines Arretierungsstiftes (41) aufweist.

12. Gelenkpfanne nach Anspruch 11, dadurch gekennzeichnet, daß jeder der Stifte (28) über seine Länge in Querrichtung in einem Abstand voneinander eine Mehrzahl von Bohrungen (40) aufweist.

13. Gelenkpfanne nach einem der Ansprüche 1 bis 10, dadurch gekennzeichnet, daß jeder der zu ihr gehörigen Stifte (28) einen mit ihm einstückigen Anschlag (41') aufweist.

14. Gelenkpfanne nach einem der Ansprüche 1 bis 13, dadurch gekennzeichnet, daß jeder der ihr zugeordneten Stifte (28) einen glatten Kopf (39) und einen Schaft (36) aufweist, der in Querrichtung Verankerungsvorsprünge (37), wie Flügel oder Widerhaken, aufweist.

15. Gelenkpfanne nach einem der Ansprüche 1 bis 14, dadurch gekennzeichnet, daß mindestens der Kopf (39) der ihr zugeordneten Stifte (28) in Querrichtung einen kreisförmigen Umriß aufweist.

16. Gelenkpfanne nach einem der Ansprüche 1 bis 15, dadurch gekennzeichnet, daß das Material der ihr zugeordneten Stifte (28) von gleicher Art wie das die Gelenkpfanne bildende Material und vorzugsweise mit diesem identisch ist.

## Claims

1. An acetabular cup for a cotyloid prosthesis, in particular for a coxofemoral prosthesis, characterised in that it has, recessed, on the convex part (19) of its outer surface at least three grooves (27), and in that it is associated with an equal number of pins (28) to be implanted into the bone cavity (14) to be fitted, in such a way that its fitting in this bone cavity (14) is effected on these pins (28) using a bayonet-type fitting.

2. An acetabular cup according to claim 1, characterised in that each of its grooves (27) comprises an inlet portion (30) which, starting from its opening on to the convex part (19) of the outer surface of the acetabular cup, extends substentially axially in the direction of the front face (21) thereof, and a common portion section (31) which, starting from said inlet portion (30), extends generally circularly while at the same time being slightly oblique in relation to said front face (21), said common portion section (31) coming closer to this front face (21) as it becomes further away from the inlet portion (30) of which it forms a continuation.

3. An acetabular cup according to claim 2, characterised in that the common portion section (31) of a groove (27) is substantially rectilinear.

4. An acetabular cup according to either of claims 2 or 3, characterised in that at its end opposite the inlet portion (30) the common portion section (31) of a groove (27) has, recessed, a widened portion (33) on that one of its flanks which is rotated axially in the direction of the front face (21) of the acetabular cup.

5. An acetabular cup according to either of claims 2 or 3, characterised in that close to its end opposite the inlet portion (30) the common portion section (31) of a groove (27) has a projecting boss (33') on at least one of its flanks.

6. An acetabular cup according to any one of claims 1 to 5, characterised in that the generators of the flanks of the common portion section (31) of its grooves (27) are substantially perpendicular to its axis.

7. An acetabular cup according to any one of claims 1 to 5, characterised in that the generators of the flanks of the common portion section (31) of its grooves (27) are oblique in relation to its axis.

8. An acetabular cup according to claim 7, characterised in that the generators of the flanks of the common portion section (31) of its grooves (27) extend substantially perpendicular to its outer surface.

9. An acetabular cup according to any one of claims 1 to 8, characterised in that it comprises three grooves (27) disposed at the vertices of an isosceles triangle whose angle (A) opposite the base (B) is less than 60°.

10. An acetabular cup according to any one of claims 1 to 9, characterised in that on its front face (21) it comprises at least one reference mark (35), the implantation of which on said front face (21) is in relation to the implantation of its grooves (27) on the convex part (19) of its outer surface.

11. An acetabular cup according to any one of claims 1 to 10, characterised in that each of the pins (28) which are associated with it has, transversally, at least one bore (4) for accommodating a stop pin (41).

12. An acetabular cup according to claim 11, characterised in that, transversally, each of the pins (28) has a plurality of bores (40) spaced apart from one another along its length.

13. An acetabular cup according to any one of claims 1 to 10, characterised in that each of the pins (28) which are associated with it has an integral stop guard (41').

14. An acetabular cup according to any one of claims 1 to 13, characterised in that each of the pins (28) which are associated with it has a smooth head (39) and a shank (36) which, transversally, is provided with anchoring projections (37), such as ribs or barbs.

15. An acetabular cup according to any one of claims 1 to 14, characterised in that the head (39) of at least the pins (28) which are associated with it has, transversally, a circular contour.

16. An acetabular cup according to any one of claims 1 to 15, characterised in that the material of the pins (28) which are associated with it is of the same type as its own constituent material and, preferably, is identical thereto.

FIG.1  FIG.5  FIG.6  FIG.7  FIG.2  FIG.3  FIG.4  FIG.8A  FIG.8B  FIG.8C  FIG.9

## FIG.10

## FIG.11

## FIG.12

11